# EUROPEAN PATENT APPLICATION

(11) **EP 4 729 105 A1**
(43) Date of publication of application: **22.04.2026**
(21) Application number: 23941666.2
(22) Date of filing: 06.10.2023
(51) Int. Cl.: A61M 25/09

(54) **MEDICAL WIRE**

(30) Priority: 14.06.2023 JP 2023097625
(71) Applicant: NHK Spring Co., Ltd., Yokohama-shi, Kanagawa 236-0004 (JP)
(72) Inventor: IMAMURA Masaru, Yokohama-shi, Kanagawa 236-0004 (JP); NAKAZONO Miho, Yokohama-shi, Kanagawa 236-0004 (JP); HAYASHIDA Hiroki, Yokohama-shi, Kanagawa 236-0004 (JP)
(74) Representative: V.O.
(86) International application number: PCT/JP2023/036564
(87) International publication number: WO 2024/257367

(57) **Abstract**

A medical wire includes a first flexible tube that extends in a front-rear direction and is formed to be bendable, an operation wire that extends in a front-rear direction and is inserted into the first flexible tube, and a support that is fixed to a rear end portion of the first flexible tube, in which a front end portion of the operation wire is fixed to a front end portion of the first flexible tube while being separated from a central axis of the first flexible tube in one direction in a radial direction.

## Description

### TECHNICAL FIELD

The present invention relates to a medical wire such as a guide wire.

Priority is claimed on Japanese Patent Application No. 2023-097625, filed June 14, 2023, the content of which is incorporated herein by reference.

### BACKGROUND ART

In the related art, as a medical wire, a guide wire has been known, as shown in, for example, Patent Document 1 below, which includes a first flexible tube (outer flexible tube 4) that extends in a front-rear direction and is formed to be bendable, an operation wire (core shaft) that extends in the front-rear direction and is inserted into the first flexible tube, and a support that is fixed to a rear end portion of the first flexible tube. In general, the guide wire is inserted into a blood vessel and is used to guide a balloon or a stent to a chronic total occlusion lesion part or the like in the blood vessel in a state where the guide wire penetrates the chronic total occlusion lesion part or the like.

In a case where the guide wire penetrates the chronic total occlusion lesion part or the like, for example, the following procedure is adopted.

First, a guide wire (first guide wire) is made to enter a blood vessel in a state where the guide wire is inserted into a microcatheter, and is made to reach a chronic total occlusion lesion part or the like in the blood vessel. The guide wire (the first guide wire) used so far is pulled out while leaving the microcatheter in the blood vessel, and then a guide wire (second guide wire) having a higher distal end load or the like than the guide wire (the first guide wire) is inserted into the microcatheter and is pierced into the chronic total occlusion lesion part or the like, so that the chronic total occlusion lesion part or the like is penetrated by the guide wire (the second guide wire) and the microcatheter. Then, the guide wire (the second guide wire) having a higher distal end load or the like is pulled out while leaving the microcatheter in the blood vessel in a state where the chronic total occlusion lesion part or the like is penetrated by the microcatheter, and the original guide wire (the first guide wire) having a lower distal end load or the like is inserted into the microcatheter again. After the guide wire (the first guide wire) has been made to reach a position where the chronic total occlusion lesion part or the like is penetrated, the microcatheter is pulled out while leaving the guide wire (the first guide wire).

### Citation List

### Patent Document

Patent Document 1: Japanese Unexamined Patent Application, First Publication No. 2011-199

### SUMMARY OF INVENTION

### Technical Problem

In a guide wire in the related art, to penetrate the chronic total occlusion lesion part or the like, it is necessary to interchangeably use guide wires having different bending stiffnesses such as distal end loads by inserting and removing the guide wires into and from the blood vessel or the like. In addition, to select a blood vessel at a blood vessel bifurcation portion and make the guide wire enter the target blood vessel until making the guide wire reach the chronic total occlusion lesion part or the like, it is necessary to insert and remove the guide wire into and from the blood vessel and finely adjust the curvature (a bending habit called pre-shape) of a front end portion of the guide wire outside the body. There has been a demand for shortening the surgical time.

The present invention provides a medical wire that can shorten the surgical time.

### Solution to Problem

A medical wire according to an aspect of the present invention includes a first flexible tube that extends in a front-rear direction and is formed to be bendable, an operation wire that extends in the front-rear direction and is inserted into the first flexible tube, and a support that is fixed to a rear end portion of the first flexible tube, in which a front end portion of the operation wire is fixed to a front end portion of the first flexible tube while being separated from a central axis of the first flexible tube in one direction in a radial direction.

The front end portion of the operation wire is fixed to the front end portion of the first flexible tube while being separated from the central axis of the first flexible tube in one direction in the radial direction. Therefore, in a case where the operation wire is pulled backward, the first flexible tube is bent in a specific one direction in the radial direction while being compressed and deformed. Accordingly, it is possible to select a blood vessel at a blood vessel bifurcation portion and make the medical wire enter the target blood vessel while operating the operation wire to bend the front end portion of the medical wire in a specific direction. Accordingly, it is possible to reduce the dependence on the operational sense of the doctor in a case where the medical wire is made to reach a chronic total occlusion lesion part or the like while being aligned with the blood vessel, and it is possible to eliminate the need for inserting and removing the medical wire into and from the blood vessel and finely adjusting the curvature of the front end portion of the medical wire outside the body.

Moreover, in a case where the operation wire is pulled backward, the front end portion of the operation wire and the front end portion of the first flexible tube are moved backward, and the first flexible tube is compressed and deformed in the front-rear direction between the first flexible tube and the support, so that the bending stiffness of the first flexible tube is increased. Therefore, the bending stiffness of the first flexible tube can be changed by operating the operation wire in a state where the medical wire is inserted into the blood vessel.

Accordingly, in a case where the medical wire penetrates a chronic total occlusion lesion part or the like, the bending stiffness of the first flexible tube can be reduced until the medical wire is made to enter a blood vessel and reach the chronic total occlusion lesion part or the like in the blood vessel, the bending stiffness of the first flexible tube can be increased by pulling the operation wire backward when the medical wire is pierced into the chronic total occlusion lesion part or the like and the medical wire penetrates the chronic total occlusion lesion part or the like, and the bending stiffness of the first flexible tube can be returned to its original low state by releasing the operation of the operation wire after the chronic total occlusion lesion part or the like is penetrated. Therefore, it is possible to eliminate the need to interchangeably use medical wires having different bending stiffnesses by inserting and removing the medical wires into and from the blood vessel or the like in order to penetrate the chronic total occlusion lesion part or the like.

As described above, it is possible to shorten the surgical time.

A bending stiffness of an intermediate portion of the first flexible tube, which is located between the front end portion and the rear end portion of the first flexible tube, may decrease from a rear side toward a front side.

The bending stiffness of the intermediate portion of the first flexible tube, which is located between the front end portion and the rear end portion, decreases from the rear side toward the front side. Therefore, only the front end portion of the medical wire can be accurately bent in a specific direction by operating the operation wire, and it is possible to select the blood vessel at the blood vessel bifurcation portion and make the medical wire enter the target blood vessel.

The medical wire may further include a second flexible tube that is inserted into the first flexible tube, extends in the front-rear direction, and is formed to be bendable, the operation wire may be inserted into the second flexible tube, a central axis of the second flexible tube and the front end portion of the operation wire may be separated from the central axis of the first flexible tube in one direction in the radial direction, a front end portion of the second flexible tube may be fixed to at least one of the front end portion of the first flexible tube and the front end portion of the operation wire, and a rear portion of the second flexible tube, which is located behind the front end portion of the second flexible tube, may be fixed to at least one of the support and the rear end portion of the first flexible tube.

The second flexible tube is provided. Therefore, in a case where the operation wire is pulled backward, the first flexible tube and the second flexible tube are bent in one direction in the radial direction while being compressed and deformed. Accordingly, for example, it is possible to easily adjust (design) a relationship between the operation force on the operation wire and the bend shape of the front end portion of the medical wire while maintaining the outer diameter of the medical wire.

A bending stiffness of an intermediate portion of the second flexible tube, which is located between the front end portion and the rear portion of the second flexible tube, may decrease from a rear side toward a front side.

Since the bending stiffness of the intermediate portion of the second flexible tube, which is located between the front end portion and the rear portion, decreases from the rear side toward the front side, only the front end portion of the medical wire can be accurately bent in a specific direction by operating the operation wire.

At least one of the first flexible tube and the second flexible tube may be a coil spring extending in the front-rear direction.

At least one of the first flexible tube and the second flexible tube is a coil spring extending in the front-rear direction. Therefore, it is possible to easily obtain a medical wire in which the front end portion can be bent in a specific direction in a case where the operation wire is pulled backward.

The medical wire may further include an operation part which is provided behind the support, to which the operation wire is fixed, and which is movable in the front-rear direction with respect to the support.

The operation part to which the operation wire is fixed is provided behind the support. Therefore, it is possible to improve the operability of the medical wire as compared with a case where the operation wire is directly operated.

### Advantageous Effects of Invention

According to the above-described aspect of the present invention, it is possible to shorten the surgical time.

### BRIEF DESCRIPTION OF DRAWINGS

[FIG. 1] A longitudinal sectional view of a medical wire according to a first embodiment.
[FIG. 2] A longitudinal sectional view of a medical wire according to a second embodiment.
[FIG. 3] A longitudinal sectional view of a medical wire according to a third embodiment.

### DESCRIPTION OF EMBODIMENTS

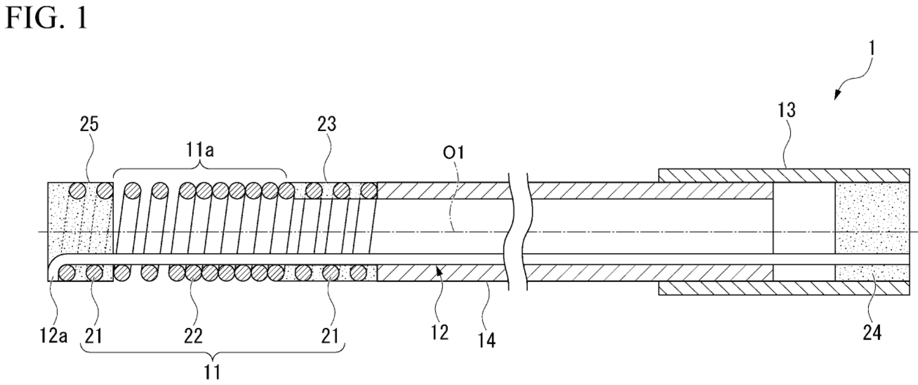

Hereinafter, a first embodiment of a medical wire will be described with reference to FIG. 1.

A medical wire 1 is a guide wire including a first flexible tube 11, an operation wire 12, an operation part 13, and a support 14. The first flexible tube 11, the operation wire 12, the operation part 13, and the support 14 are formed of, for example, metal materials or the like. The materials forming the first flexible tube 11, the operation wire 12, the operation part 13, and the support 14 may be appropriately changed. The operation part 13 may not be provided.

The operation part 13 and the support 14 are formed in a tubular shape and are disposed coaxially with a central axis O1 of the first flexible tube 11.

Hereinafter, a side on which the first flexible tube 11 is located with respect to the support 14 in a direction in which the central axis O1 extends is referred to as a front side, a side on which the operation part 13 is located with respect to the support 14 in the direction in which the central axis O1 extends is referred to as a rear side, and a direction in which the central axis O1 extends is referred to as a front-rear direction. A direction intersecting the central axis O1 as viewed from the front-rear direction is referred to as a radial direction, and a direction going around the central axis O1 as viewed from the front-rear direction is referred to as a circumferential direction.

The first flexible tube 11 extends in the front-rear direction and is formed to be bendable. The first flexible tube 11 is a coil spring extending in the front-rear direction.

The outer diameter of the first flexible tube 11 is set to a size that allows the first flexible tube 11 to be inserted into a blood vessel (for example, 0.2 mm or more and 1.0 mm or less). The outer shape and the size of the first flexible tube 11 as viewed in the front-rear direction are the same over the entire length in the front-rear direction. The diameter of a wire material that forms the first flexible tube 11 is the same over the entire length.

The diameter of the wire material that forms the first flexible tube 11 may not be the same over the entire length. In addition, the outer shape and the size of the first flexible tube 11 as viewed in the front-rear direction may not be the same over the entire length in the front-rear direction. For example, the outer diameter of the first flexible tube 11 may decrease toward the front. The first flexible tube 11 is not limited to the coil spring, and may be, for example, a tube or the like having a peripheral wall that continuously extends over the entire length in each of the front-rear direction and the circumferential direction.

The support 14 is fixed to a rear end portion of the first flexible tube 11. The support 14 extends in the front-rear direction and is formed to be bendable. A rear end opening edge of the first flexible tube 11 abuts against a front end opening edge of the support 14 in the front-rear direction. A front end portion of the support 14 and the rear end portion of the first flexible tube 11 are fixed by, for example, soldering, brazing, adhesion, crimping, or the like.

In the example shown in the drawing, for example, a tubular middle fixing member 23 made of a solder material, a brazing material, an adhesive, or the like extends forward from the front end opening edge of the support 14. The wall thickness of the middle fixing member 23 is equal to the diameter of the wire material that forms the first flexible tube 11, which is a coil spring. The rear end portion of the first flexible tube 11 is embedded in the middle fixing member 23 in a state in which a rear end opening portion of the first flexible tube 11 is open. An outer peripheral surface of the middle fixing member 23 is connected to an outer peripheral surface of the support 14 and an outer peripheral surface of the first flexible tube 11 in the front-rear direction without a step, and an inner peripheral surface of the middle fixing member 23 is connected to an inner peripheral surface of the support 14 and an inner peripheral surface of the first flexible tube 11 in the front-rear direction without a step.

The outer diameter of the support 14 is set to be equal to the outer diameter of the first flexible tube 11 and is set to have such a size that the support 14 can be inserted into a blood vessel (for example, 0.2 mm or more and 1.0 mm or less). The outer shape and the size of the support 14 as viewed in the front-rear direction are the same over the entire length in the front-rear direction.

The outer shape and the size of the support 14 as viewed in the front-rear direction may not be the same over the entire length in the front-rear direction. For example, the outer diameter of the support 14 may decrease toward the front. The outer shapes and the sizes of the support 14 and the first flexible tube 11 as viewed in the front-rear direction may be different from each other. In this case, it is preferable that the support 14 does not protrude outward in the radial direction from the outer peripheral surface of the first flexible tube 11 as viewed from the front-rear direction.

The operation part 13 is provided behind the support 14. The operation part 13 protrudes backward from the support 14. The operation part 13 is provided to be movable in the front-rear direction with respect to the support 14. In the example shown in the drawing, a rear end portion of the support 14 is inserted into a front portion of the operation part 13.

A front end opening edge of the operation part 13 and a rear end opening edge of the support 14 may face each other in the front-rear direction, and the front portion of the operation part 13 may be inserted into the rear end portion of the support 14.

A rear portion of the operation wire 12 is fixed to the operation part 13 by, for example, soldering, brazing, adhesion, crimping, or the like. In the example shown in the drawing, a rear end opening portion of the operation part 13 is closed by a rear fixing member 24 made of, for example, a solder material, a brazing material, an adhesive, or the like, and the rear portion of the operation wire 12 is embedded in the rear fixing member 24.

The operation wire 12 extends in the front-rear direction and is inserted into the first flexible tube 11. The operation wire 12 is formed to be elastically deformable. A front end portion of the operation wire 12 is fixed to a front end portion of the first flexible tube 11 by, for example, soldering, brazing, adhesion, crimping, or the like while being separated from the central axis O1 in one direction in the radial direction.

A plurality of the operation wires 12 may be provided at intervals in the circumferential direction.

The operation wire 12 extends straight in the front-rear direction. The operation wire 12 may extend in the front-rear direction while bending or the like, for example. The operation wire 12 is disposed on the inner peripheral surface of the first flexible tube 11 and on the inner peripheral surface of the support 14. The rear portion of the operation wire 12 protrudes backward from the support 14. A protruding portion 12a of the front end portion of the operation wire 12, which protrudes forward from the first flexible tube 11, is bent in one direction in the radial direction and is locked to a front end opening edge of the first flexible tube 11. The protruding portion 12a of the operation wire 12 is located at the same position in the radial direction or on the inner side in the radial direction with respect to the outer peripheral surface of the front end portion of the first flexible tube 11. A distal end surface of the operation wire 12 faces one direction in the radial direction.

In the example shown in the drawing, a front end opening portion of the first flexible tube 11 is closed by a front fixing member 25 made of, for example, a solder material, a brazing material, an adhesive, or the like. An outer peripheral surface of the front fixing member 25 is connected to the outer peripheral surface of the first flexible tube 11 in the front-rear direction without a step. The front end portion of the operation wire 12 including the protruding portion 12a is embedded in the front fixing member 25.

The bending stiffness of an intermediate portion 11a of the first flexible tube 11, which is located between the front end portion (front fixing member 25) and the rear end portion (middle fixing member 23), decreases from the rear side toward the front side.

In the example shown in the drawing, the first flexible tube 11 includes a first rigid tube portion 22 having high bending stiffness and a pair of first flexible tube portions 21 having lower bending stiffness than the first rigid tube portion 22 and sandwiching the first rigid tube portion 22 therebetween in the front-rear direction. In the pair of first flexible tube portions 21, the number of times of winding of a front first flexible tube portion 21 located on the front side is larger than the number of times of winding of a rear first flexible tube portion 21 located on the rear side. The number of times of winding of the front first flexible tube portion 21 may be equal to or less than the number of times of winding of the rear first flexible tube portion 21.

The first flexible tube portion 21 is formed such that an interval (wire material gap) between the wire materials adjacent to each other in the front-rear direction is larger than that of the first rigid tube portion 22. Accordingly, the bending stiffness of the first flexible tube portion 21 is smaller than the bending stiffness of the first rigid tube portion 22, and the first flexible tube portion 21 is flexibly bent following the bent blood vessel.

In the example shown in the drawing, the wire materials adjacent to each other in the front-rear direction in the first rigid tube portion 22 abut against each other. That is, the first rigid tube portion 22 is a tightly wound coil spring. Accordingly, in the intermediate portion 11a of the first flexible tube 11, the first rigid tube portion 22 can be flexibly bent following the bending deformation of the first flexible tube portion 21.

A front portion of the front first flexible tube portion 21 of the pair of first flexible tube portions 21, which is located on the front side, is joined to or adhered to the front fixing member 25, and a rear portion of the front first flexible tube portion 21 is located between the front fixing member 25 and the middle fixing member 23. The rear first flexible tube portion 21 of the pair of first flexible tube portions 21, which is located on the rear side, is joined or adhered to the middle fixing member 23 over the entire region.

Therefore, the intermediate portion 11a of the first flexible tube 11, which is located between the front fixing member 25 and the middle fixing member 23, is composed of the entire first rigid tube portion 22 and the rear portion of the front first flexible tube portion 21 of the pair of first flexible tube portions 21, which is located on the front side. Accordingly, the bending stiffness of the intermediate portion 11a of the first flexible tube 11 gradually decreases from the rear side toward the front side. The bending stiffness of the intermediate portion 11a of the first flexible tube 11 may linearly decrease from the rear side toward the front side.

In the example shown in the drawing, the length of the first rigid tube portion 22 in the front-rear direction is longer than the length, in the front-rear direction, of the rear portion of the front first flexible tube portion 21 of the pair of first flexible tube portions 21, which is located on the front side. The length of the first rigid tube portion 22 in the front-rear direction may be equal to or less than the length of the rear portion of the front first flexible tube portion 21 in the front-rear direction.

As described above, according to the medical wire 1 of the present embodiment, the front end portion of the operation wire 12 is fixed to the front end portion of the first flexible tube 11 while being separated from the central axis O1 of the first flexible tube 11 in one direction in the radial direction. Therefore, in a case where the operation wire 12 is pulled backward, the first flexible tube 11 is bent in one direction in the radial direction while being compressed and deformed. Accordingly, it is possible to select a blood vessel at a blood vessel bifurcation portion and make the medical wire 1 enter the target blood vessel while operating the operation wire 12 to bend the front end portion of the medical wire 1 in a desired direction. Accordingly, it is possible to reduce the dependence on the operational sense of the doctor in a case where the medical wire 1 is made to reach a chronic total occlusion lesion part or the like while being aligned with the blood vessel, and it is possible to eliminate the need for inserting and removing the medical wire into and from the blood vessel and finely adjusting the curvature of the front end portion of the medical wire outside the body.

Moreover, in a case where the operation wire 12 is pulled backward completely, the front end portion of the operation wire 12 and the front end portion of the first flexible tube 11 are moved backward, and the first flexible tube 11 is compressed and deformed in the front-rear direction between the first flexible tube 11 and the support 14, so that the bending stiffness of the first flexible tube 11 is increased. Therefore, the bending stiffness of the first flexible tube 11 can be changed by operating the operation wire 12 in a state in which the medical wire 1 is inserted into the blood vessel.

Accordingly, in a case where the medical wire 1 penetrates a chronic total occlusion lesion part or the like, the bending stiffness of the first flexible tube 11 can be reduced until the medical wire 1 is made to enter a blood vessel and reach the chronic total occlusion lesion part or the like in the blood vessel, the bending stiffness of the first flexible tube 11 can be increased by pulling the operation wire 12 backward when the medical wire 1 is pierced into the chronic total occlusion lesion part or the like and the medical wire 1 penetrates the chronic total occlusion lesion part or the like, and the bending stiffness of the first flexible tube 11 can be returned to its original low state by releasing the operation of the operation wire 12 after the chronic total occlusion lesion part or the like is penetrated. Therefore, it is possible to eliminate the need to interchangeably use medical wires having different bending stiffnesses by inserting and removing the medical wires into and from the blood vessel or the like in order to penetrate the chronic total occlusion lesion part or the like.

As described above, it is possible to shorten the surgical time.

The bending stiffness of the intermediate portion 11a of the first flexible tube 11, which is located between the front end portion (front fixing member 25) and the rear end portion (middle fixing member 23), decreases from the rear side toward the front side. Therefore, the front end portion of the medical wire 1 can be accurately bent in a desired direction by operating the operation wire 12, and it is possible to select the blood vessel at the blood vessel bifurcation portion and make the medical wire 1 easily enter the target blood vessel.

The operation part 13 to which the operation wire 12 is fixed is provided behind the support 14. Therefore, it is possible to improve the operability of the medical wire 1 as compared with a case where the operation wire 12 is directly operated.

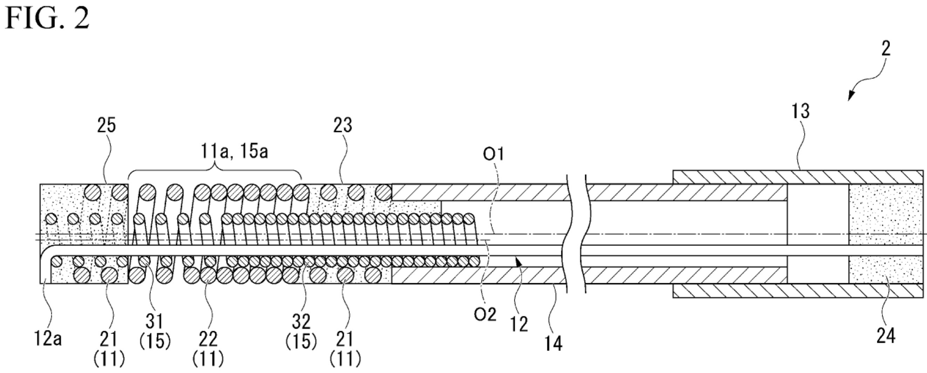

Next, a medical wire 2 according to a second embodiment of the present invention will be described with reference to FIG. 2.

In the second embodiment, the same portions as the components in the first embodiment will be designated by the same reference signs, and a description thereof will be omitted, and only different points will be described.

In the medical wire 2 of the present embodiment, a second flexible tube 15 that extends in the front-rear direction and is formed to be bendable is inserted into the first flexible tube 11. A front end portion of the second flexible tube 15 is fixed to at least one of the front end portion of the first flexible tube 11 and the front end portion of the operation wire 12. A rear portion of the second flexible tube 15, which is located behind the front end portion, is fixed to at least one of the support 14 and the rear end portion of the first flexible tube 11.

The second flexible tube 15 protrudes from the first flexible tube 11 in both forward and backward in the front-rear direction. The second flexible tube 15 may not protrude from the first flexible tube 11 in the front-rear direction.

At least one of the first flexible tube 11 and the second flexible tube 15 is a coil spring extending in the front-rear direction. In the example shown in the drawing, both the first flexible tube 11 and the second flexible tube 15 are coil springs extending in the front-rear direction. The winding directions of the first flexible tube 11 and the second flexible tube 15 are opposite to each other.

The winding directions of the first flexible tube 11 and the second flexible tube 15 may be the same as each other. Any one of the first flexible tube 11 and the second flexible tube 15 is not limited to the coil spring, and may be, for example, a tube having a peripheral wall that continuously extends over the entire length in each of the front-rear direction and the circumferential direction.

The bending stiffness of an intermediate portion 15a of the second flexible tube 15, which is located between the front end portion (front fixing member 25) and the rear portion (middle fixing member 23), decreases from the rear side toward the front side.

In the example shown in the drawing, the second flexible tube 15 is configured such that a second flexible tube portion 31 having lower bending stiffness and a second rigid tube portion 32 having higher bending stiffness than the second flexible tube portion 31 are connected in this order from the front toward the rear. The second flexible tube portion 31 protrudes forward from the first flexible tube 11, and the second rigid tube portion 32 protrudes backward from the first flexible tube 11. The length of the second flexible tube portion 31 in the front-rear direction is slightly shorter than the length of the second rigid tube portion 32 in the front-rear direction.

The second flexible tube portion 31 is formed such that an interval (wire material gap) between wire materials adjacent to each other in the front-rear direction is larger than that of the second rigid tube portion 32. Accordingly, the bending stiffness of the second flexible tube portion 31 is smaller than the bending stiffness of the second rigid tube portion 32, and the second flexible tube portion 31 is flexibly bent following the bent blood vessel.

In the example shown in the drawing, the wire materials adjacent to each other in the front-rear direction in the second rigid tube portion 32 abut against each other. That is, the second rigid tube portion 32 is a tightly wound coil spring. Accordingly, in the intermediate portion 15a of the second flexible tube 15, the second rigid tube portion 32 can be flexibly bent following the bending deformation of the second flexible tube portion 31.

A front portion of the second flexible tube portion 31 is joined to or adhered to the front fixing member 25. Accordingly, the front end portion of the second flexible tube 15 is fixed to the front end portion of the first flexible tube 11 and the front end portion of the operation wire 12 via the front fixing member 25. A rear portion of the second flexible tube portion 31 is located between the front fixing member 25 and the middle fixing member 23.

The middle fixing member 23 is joined or adhered to an outer peripheral surface of a portion of the second rigid tube portion 32, which is located between a front end portion of the second rigid tube portion 32 located inside the first flexible tube 11 and a rear end portion of the second rigid tube portion 32 located inside the support 14. Accordingly, the rear portion of the second flexible tube 15, which is located behind the front end portion, is fixed to the support 14 and the rear end portion of the first flexible tube 11 via the middle fixing member 23. In the example shown in the drawing, the middle fixing member 23 does not block the inside of the second rigid tube portion 32, and the inside of the support 14 communicates with the inside of the rear portion of the second flexible tube portion 31 through the inside of the second rigid tube portion 32.

The intermediate portion 15a of the second flexible tube 15, which is located between the front fixing member 25 and the middle fixing member 23, is composed of the rear portion of the second flexible tube portion 31 and the front end portion of the second rigid tube portion 32. Accordingly, the bending stiffness of the intermediate portion 15a of the second flexible tube 15 gradually decreases from the rear side toward the front side. The bending stiffness of the intermediate portion 15a of the second flexible tube 15 may linearly decrease from the rear side toward the front side.

In the example shown in the drawing, the length of the rear portion of the second flexible tube portion 31 in the front-rear direction is longer than the length of the front end portion of the second rigid tube portion 32 in the front-rear direction. The length of the rear portion of the second flexible tube portion 31 in the front-rear direction may be equal to or less than the length of the front end portion of the second rigid tube portion 32 in the front-rear direction. The rear portion of the second flexible tube portion 31 straddles, in the front-rear direction, a boundary portion between the first rigid tube portion 22 and the front first flexible tube portion 21 of the pair of first flexible tube portions 21, which is located on the front side, in the intermediate portion 11a of the first flexible tube 11.

The operation wire 12 is inserted into the second flexible tube 15. A central axis O2 of the second flexible tube 15 and the front end portion of the operation wire 12 are separated from the central axis O1 of the first flexible tube 11 in one direction in the radial direction.

The front end portion of the operation wire 12 is fixed to the front end portion of the second flexible tube 15 by, for example, soldering, brazing, adhesion, crimping, or the like while being separated from the central axis O2 of the second flexible tube 15 in one direction in the radial direction. The operation wire 12 is disposed on an inner peripheral surface of the second flexible tube 15.

The protruding portion 12a of the front end portion of the operation wire 12, which protrudes forward from the second flexible tube 15, is bent in one direction in the radial direction and is locked to a front end opening edge of the second flexible tube 15. The protruding portion 12a of the operation wire 12 is located at the same position in the radial direction or on the inner side in the radial direction with respect to the outer peripheral surface of the front end portion of the first flexible tube 11. The protruding portion 12a of the operation wire 12 is separated forward from the front end opening edge of the first flexible tube 11 and faces the front end opening edge of the first flexible tube 11 the front-rear direction. A region between the protruding portion 12a of the operation wire 12 and the front end opening edge of the first flexible tube 11 is also filled with the front fixing member 25, and the front end portion of the operation wire 12 including the protruding portion 12a is embedded in the front fixing member 25. The distal end surface of the operation wire 12 faces one direction in the radial direction.

As described above, the medical wire 2 according to the present embodiment also has the same effects as the medical wire 1 shown in FIG. 1.

The second flexible tube 15 is provided. Therefore, in a case where the operation wire 12 is pulled backward, the first flexible tube 11 and the second flexible tube 15 are bent in one direction in the radial direction while being compressed and deformed. Accordingly, for example, it is possible to easily adjust (design) a relationship between the operation force on the operation wire 12 and the bend shape of the front end portion of the medical wire 2 while maintaining the outer diameter of the medical wire 2.

The bending stiffness of the intermediate portion 15a of the second flexible tube 15, which is located between the front end portion (front fixing member 25) and the rear portion (middle fixing member 23), decreases from the rear side toward the front side. Therefore, the front end portion of the medical wire 2 can be accurately bent in a desired direction by operating the operation wire 12.

At least one of the first flexible tube 11 and the second flexible tube 15 is a coil spring extending in the front-rear direction. Therefore, it is possible to easily obtain the medical wire 2 in which the front end portion can be bent in a desired direction in a case where the operation wire 12 is pulled backward.

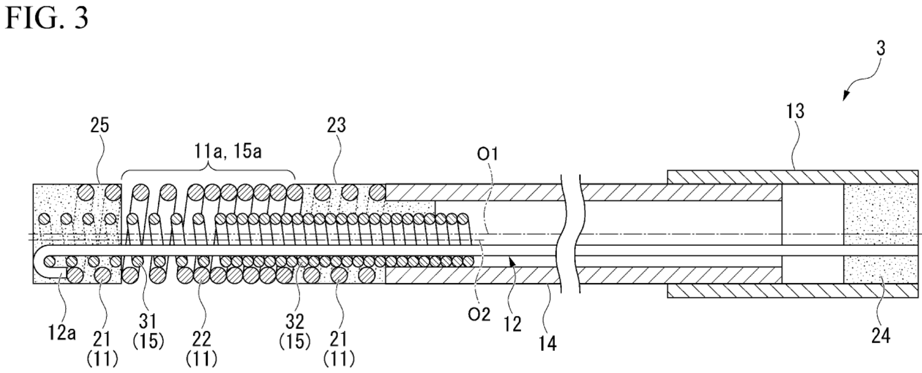

Next, a medical wire 3 according to a third embodiment of the present invention will be described with reference to FIG. 3.

In the third embodiment, the same portions as the components in the second embodiment will be designated by the same reference signs, and a description thereof will be omitted, and only different points will be described.

In the medical wire 3 of the present embodiment, the protruding portion 12a of the front end portion of the operation wire 12, which protrudes forward from the second flexible tube 15, is folded backward while being bent in one direction in the radial direction, and is locked to the front end opening edge of the second flexible tube 15. A part of the front end portion of the second flexible tube 15 in the circumferential direction is sandwiched in the radial direction by the front end portion of the operation wire 12. The distal end surface of the operation wire 12 faces backward. The distal end surface of the operation wire 12 abuts against or is close to the front end opening edge of the first flexible tube 11.

As described above, the medical wire 3 according to the present embodiment also has the same effects as the medical wire 2 shown in FIG. 2.

In addition, the technical scope of the present invention is not limited to the above embodiment, and various changes can be made without departing from the spirit of the present invention.

For example, the bending stiffness of the intermediate portions 11a and 15a may be the same over the entire length in the front-rear direction.

In addition, it is possible to appropriately replace the components in the above embodiments with well-known components without departing from the spirit of the present invention. Additionally, the above-described embodiments and the modification examples may be combined with each other as appropriate.

The aspect of the present invention is, for example, as follows.
<1> A medical wire including a first flexible tube that extends in a front-rear direction and is formed to be bendable, an operation wire that extends in the front-rear direction and is inserted into the first flexible tube, and a support that is fixed to a rear end portion of the first flexible tube, in which a front end portion of the operation wire is fixed to a front end portion of the first flexible tube while being separated from a central axis of the first flexible tube in one direction in a radial direction.
<2> The medical wire according to the above <1>, in which a bending stiffness of an intermediate portion of the first flexible tube, which is located between the front end portion and the rear end portion of the first flexible tube, decreases from a rear side toward a front side.
<3> The medical wire according to the above <1> or <2>, further including a second flexible tube that is inserted into the first flexible tube, extends in the front-rear direction, and is formed to be bendable, in which the operation wire is inserted into the second flexible tube, a central axis of the second flexible tube and the front end portion of the operation wire are separated from the central axis of the first flexible tube in one direction in the radial direction, a front end portion of the second flexible tube is fixed to at least one of the front end portion of the first flexible tube and the front end portion of the operation wire, and a rear portion of the second flexible tube, which is located behind the front end portion of the second flexible tube, is fixed to at least one of the support and the rear end portion of the first flexible tube.
<4> The medical wire according to the above <3>, in which a bending stiffness of an intermediate portion of the second flexible tube, which is located between the front end portion and the rear portion of the second flexible tube, decreases from a rear side toward a front side.
<5> The medical wire according to the above <3> or <4>, in which at least one of the first flexible tube and the second flexible tube is a coil spring extending in the front-rear direction.
<6> The medical wire according to any one of the above <1> to <5>, further including an operation part which is provided behind the support, to which the operation wire is fixed, and which is movable in the front-rear direction with respect to the support.

### INDUSTRIAL APPLICABILITY

According to the present invention, it is possible to provide a medical wire that can shorten the surgical time.

### REFERENCE SIGNS LIST

1, 2, 3 Medical wire
11 First flexible tube
11a Intermediate portion of first flexible tube
12 Operation wire
13 Operation part
14 Support
15 Second flexible tube
15a Intermediate portion of second flexible tube
O1 Central axis of first flexible tube
O2 Central axis of second flexible tube

## Claims

1. A medical wire comprising:
a first flexible tube that extends in a front-rear direction and is formed to be bendable;
an operation wire that extends in the front-rear direction and is inserted into the first flexible tube; and
a support that is fixed to a rear end portion of the first flexible tube,
wherein a front end portion of the operation wire is fixed to a front end portion of the first flexible tube while being separated from a central axis of the first flexible tube in one direction in a radial direction.

2. The medical wire according to Claim 1,
wherein a bending stiffness of an intermediate portion of the first flexible tube, which is located between the front end portion and the rear end portion of the first flexible tube, decreases from a rear side toward a front side.

3. The medical wire according to Claim 1 or 2, further comprising:
a second flexible tube that is inserted into the first flexible tube, extends in the front-rear direction, and is formed to be bendable,
wherein the operation wire is inserted into the second flexible tube,
a central axis of the second flexible tube and the front end portion of the operation wire are separated from the central axis of the first flexible tube in one direction in the radial direction,
a front end portion of the second flexible tube is fixed to at least one of the front end portion of the first flexible tube and the front end portion of the operation wire, and
a rear portion of the second flexible tube, which is located behind the front end portion of the second flexible tube, is fixed to at least one of the support and the rear end portion of the first flexible tube.

4. The medical wire according to Claim 3,
wherein a bending stiffness of an intermediate portion of the second flexible tube, which is located between the front end portion and the rear portion of the second flexible tube, decreases from a rear side toward a front side.

5. The medical wire according to Claim 3,
wherein at least one of the first flexible tube and the second flexible tube is a coil spring extending in the front-rear direction.

6. The medical wire according to Claim 1 or 2, further comprising:
an operation part which is provided behind the support, to which the operation wire is fixed, and which is movable in the front-rear direction with respect to the support.
